# EUROPEAN PATENT APPLICATION

(11) **EP 3 736 816 A1**
(43) Date of publication of application: **11.11.2020**
(21) Application number: 19172897.1
(22) Date of filing: 07.05.2019
(51) Int. Cl.: G16C 20/30, G16C 20/70

(54) **DRUG CANDIDATE SAFETY PREDICTIONS**

(71) Applicant: Bayer AG, 51373 Leverkusen (DE)
(72) Inventor: JENSEN, Markus, 50226 Frechen (DE); BRAUN, Inka Valeska, 50670 Köln (DE); SCHLUETER, Thorsten, 58332 Schwelm (DE); DE ZOETE, Jacob Coenraad, 12169 Berlin (DE)
(74) Representative: BIP Patents

(57) **Abstract**

The present invention deals with the prediction of a safety score for a drug candidate. The present invention provides a method, a computer system, and a computer program product for the prediction of a safety score for a drug candidate.

## Description

The present invention deals with the prediction of a safety score for a drug candidate. The present invention provides a method, a computer system, and a computer program product for the prediction of a safety score for a drug candidate.

Drug safety has been recognized as one of the main causes of attrition during clinical trials. Therefore, drug safety should be evaluated extensively as early as possible. Usually, *in vitro* and *in vivo* tests are performed to investigate drug safety, including a variety of toxicities and adverse drug effects.

In recent years, there are also some efforts to develop *in vitro* models such as "organ on a chip" to reduce cost. However, those approaches are still costly and time-consuming.

In comparison of experimental approaches, computational methods have shown great advantages since they are fast, cheap, accurate, and most importantly they can be done before a compound is synthesized.

Earlier methods for computational pre-screening focused primarily on chemical features of potential compounds. The first approaches were frequently based on rule sets with scores awarded to compounds for not failing particular criteria of "druglikeness".

From the pharmacokinetic perspective, it was proposed to characterize compounds according to absorption, distribution, metabolism, and excretion criteria (ADME).

Further developments have led to refinements of simple rule-based methods into more granular qualitative measures.

WO2018/049376 discloses a data-driven computational system for predicting the likelihood of toxicity in clinical trials and whether the drug would pass the toxicity requirements of a clinical trial. The system uses properties of a chemical's gene targets and chemical structure to provide a toxicity predictor score.

Practice has demonstrated that for many drug candidates "toxicity" is not an adequate criterion variable. In addition, the system disclosed in WO2018/049376 requires information about one or more gene targets for toxicity prediction which are sometimes not available or require some experimental efforts.

Proceeding from the prior art, the technical problem is therefore that of providing simple, easy-to-use and reliable means which generate a prediction of the probability that a drug candidate will successfully pass a clinical trial.

This problem is solved by the subjects of the independent claims. Preferred embodiments are found in the dependent claims and in the present description and the drawings.

In a first aspect, the present invention provides a method comprising the steps of:
- receiving a chemical structure of a test chemical compound,
- determining a plurality of predictor values from the chemical structure of the test chemical compound,
- generating a feature vector from the plurality of predictor values
- supplying the feature vector to a machine learning prediction model, the machine learning prediction model being trained with a first plurality of feature vectors and a second plurality of feature vectors, the first plurality of feature vectors being determined from chemical structures of reference chemical compounds which have failed in a non-oncological clinical trial due to a safety issue, the second plurality of feature vectors being determined from chemical structures of reference chemical compounds which passed a non-oncological clinical trial without any safety issue,
- receiving from the machine learning prediction model a safety score, the safety score representing a probability of the test chemical compound failing in a non-oncological clinical trial due to a safety issue.
- outputting the safety score.

The present invention provides, in a second aspect, a computer system comprising
- an input unit,
- one or more predictor value generation unit(s),
- a feature vector generation unit,
- a prediction unit, and
- an output unit,
   wherein the input unit is configured to receive a chemical structure of a test chemical compound, wherein the one or more predictor value generation unit(s) is/are configured to determine a plurality of predictor values from the chemical structure of the test chemical compound,
   wherein the feature vector generation unit is configured to generate a feature vector from the plurality of predictor values,
   wherein the prediction unit is configured to determine a safety score from the feature vector using a machine learning prediction model,
   - the machine learning prediction model being trained with a first plurality of feature vectors and a second plurality of feature vectors, the first plurality of feature vectors being determined from chemical structures of reference chemical compounds which have failed in a non-oncological clinical trial due to a safety issue, the second plurality of feature vectors being determined from chemical structures of reference chemical compounds which passed a non-oncological clinical trial without any safety issue,
   - the safety score representing a probability of the test chemical compound failing in a non-oncological clinical trial due to a safety issue.
wherein the output unit is configured to output the safety score.

The present invention further provides, in a third aspect, a computer program product comprising program code which can be loaded into a memory of a computer and causes the computer to execute the following steps:
- receiving a chemical structure of a test chemical compound,
- determining a plurality of predictor values from the chemical structure of the test chemical compound,
- generating a feature vector from the plurality of predictor values
- supplying the feature vector to a machine learning prediction model, the machine learning prediction model being trained with a first plurality of feature vectors and a second plurality of feature vectors, the first plurality of feature vectors being determined from chemical structures of reference chemical compounds which have failed in a non-oncological clinical trial due to a safety issue, the second plurality of feature vectors being determined from chemical structures of reference chemical compounds which passed a non-oncological clinical trial without any safety issue,
- receiving from the machine learning prediction model a safety score, the safety score representing a probability of the test chemical compound failing in a non-oncological clinical trial due to a safety issue.
- outputting the safety score.

The invention will be more particularly elucidated below without distinguishing between the subjects of the invention (method, computer system, computer program product). On the contrary, the following elucidations are intended to apply analogously to all subjects of the invention, irrespective of in which context they occur.

When the present description or the claims mention steps in an order, this does not necessarily mean that the invention is restricted to the order mentioned. On the contrary, it is conceivable for the steps to be carried out in a different order as well, or in parallel to one another as well; an exception is when one step builds on another step, this making it absolutely necessary for the building step to be carried out in a successive manner (this being clear, however, in individual cases). The orders mentioned are thus preferred embodiments of the invention.

The present invention serves to predict a safety score for a test chemical compound. The safety score represents a probability of the test chemical compound failing in a non-oncological clinical trial due to a safety issue.

Drug safety is a more appropriate criterion variable for predicting the druglikeness of a chemical compound than toxicity because it also includes further undesired clinical effects (on target effects, allergic reactions, etc.). Another advantage of drug safety compared to toxicity is the possibility to use the prediction outcome for optimally shaping the clinical investigation program at an early stage of drug development. Furthermore, safety is a clinical surrogate endpoint.

The present invention generates a safety score for a test chemical compound on the basis of the results obtained from a variety of different drug candidates used in past clinical trials.

Clinical trials are experiments or observations done in clinical research. Such prospective biomedical research studies on human participants are designed to answer specific questions about new drugs. Clinical trials generate data on safety and efficacy.

Clinical trials of new drugs are usually described based on their phase. The FDA (Food and Drug Administration) typically requires phase I, II, and III trials to be conducted to determine if the drug can be approved for use.

A phase I trial tests an experimental treatment on a small group of often healthy people to judge its safety and side effects and to find the correct drug dosage.

While the emphasis in phase I is on safety, the emphasis in phase II is on effectiveness. This phase aims to obtain preliminary data on whether the drug works in people who have a certain disease or condition. These trials also continue to study safety, including short-term side effects.

A phase III trial gathers more information about safety and effectiveness, studying different populations and/or different dosages and/or using the drug in combination with other drugs.

If an authority such as the FDA or the EMA (European Medicines Agency) agrees that the trial results are positive, it will approve the experimental drug.

A phase IV trial for drugs takes place after the authority approves their use. A drug's effectiveness and safety are monitored in large, diverse populations. Sometimes, the side effects of a drug may not become clear until more people have taken it over a longer period of time.

The present invention can use information from all phases of clinical research (phase I, II, III, and/or IV). In a preferred embodiment, the prediction model is built from information obtained in phase I, phase II, and phase III clinical trials.

Both, safety and efficacy are evaluated in a clinical trial relative to how the treatment is intended to be used, what other treatments are available, and the severity of the disease or condition. The benefits must outweigh the risks. Nevertheless, drug development does differ depending on the indication the drug is developed in. Looking at oncological drugs to treat cancer, severe side effects are accepted that would not be acceptable e.g. for an over-the-counter pain medication, yet the cancer drugs have been approved since they are used under a physician's care, and are used for a life-threatening condition.

Therefore, according to the present invention, only non-oncological clinical trials are taken into account for the prediction of a safety score.

An oncological clinical trial is a clinical trial in which a new drug for the treatment of an oncology disorder (cancer) is evaluated.

Excluding oncological clinical trials from the prediction of a safety score, as described herein, results in a higher accuracy of the predicted outcome.

In a preferred embodiment, the safety of a drug is classified into at least two categories: safe and unsafe. A chemical compound is considered to be safe if it passed one or more non-oncological clinical trials without any safety issue. A chemical compound is considered to be unsafe if it failed in a non-oncological clinical trial because of a safety issue.

In another preferred embodiment, chemical compounds which failed in a phase I clinical trial, in a phase II clinical trial, or in a phase III clinical trial due to a safety issue form a first plurality of reference chemical compounds which can be considered as unsafe; chemical compounds which passed phases I to III and which were approved by an authority like the FDA or the EMA form a second plurality of reference chemical compounds which are considered as safe.

Other definitions of safety of reference chemical compounds are conceivable.

It is for example conceivable to form four classes of safety: a first class can be formed by reference chemical compounds which failed in a phase I clinical trial due to a safety issue; a second class can be formed by reference chemical compounds which passed a phase I clinical trial but failed in a phase II clinical trial; a third calls can be formed by reference chemical compounds which passed phases I and II but which failed in a phase III clinical trial; and a fourth class can be formed by reference chemical compounds which passed phases I to III.

From the results of past non-oncological clinical trials, a prediction model is built which links structural features of reference chemical compounds to their safety properties.

In order to do this, for each reference chemical compound which is used for building (training) the prediction model, a feature vector is created.

In general, feature vectors are used to represent numeric or symbolic characteristics, called features, of an object in a mathematical, easily analyzable way. They are important for many different areas of machine learning and pattern processing. Machine learning algorithms typically require a numerical representation of objects in order for the algorithms to do processing and (statistical) analysis.

The procedure of generating a feature vector from the chemical structure of a chemical compound can be done in a three-step approach. In a first step, a plurality of descriptors is determined from the chemical structure of the chemical compound. In a second step, predictor values are generated from the descriptors. In a third step, the predictor values are merged into a feature vector.

The generation of descriptors from a chemical structure of a chemical compound is known e.g. from QSAR (Quantitative Structure Activity Relationship) studies.

Examples of descriptors are the molecular weight, number of aromatic atoms, number of hydrophobic atoms, number of heavy atoms, total number of atoms, van der Waals accessible surface area of hydrophobic atoms, polar surface area, hydrogen bond donor and acceptor counts, formal charge, number of rotatable bounds (non-aromatic bond, non-double bond), Lipinski's Rule of Five feature, octanol-water partition coefficient (e.g. as logP: logarithm of the equilibrium concentration in octanol-water), and the like.

The following publications provide an overview of descriptors derived from the chemical structure of a chemical compound which can be used as predictors for predicting a safety score: Danishuddin and Asad U. Khan: Descriptors and their selection methods in QSAR analysis: paradigm for drug design, Drug Discovery Today, Volume 21, Number 8, August 2016, Elsevier; Yu-Chen Lo et al.: Machine learning in cheminformatics and drug discovery, Drug Discovery Today, Vol. 23, No. 8, August 2018, Elesevier; J. Leszczynski et al.: Challenges and advantages in computational chemistry and physics Vol. 8 Recent Advances in QSAR Studies, Springer 2010, DOI 10.1007/978-1-4020-9783-6; http://qsar.sourceforge.net/dicts/qsar-descriptors/index.xhtml; Todeschini R. & Consonni V., Handbook of Molecular Descriptors, J. Wiley & Sons, New York, 2008, 688 pp; Karelson M., Molecular Descriptors in QSAR/QSPR, J. Wiley & Sons, New York, 2000, 430 pp; Basak S. C., Grunwald G. D. & Niemi G. J., Use of Graph Theoretical and Geometrical Molecular Descriptors in Structure-Activity Relationships, in From Chemical Topology to Three-Dimensional Geometry, Plenum Press, New York, 1997, pp. 73-116; Benfenati E., Casalegno M., Cotterill J., Price N., Spreafico M., and Toropov A., Characterization of chemical structures, in: Benfenati E. (Ed.), Quantitative Structure-Activity Relationships (QSAR) for Pesticide Regulatory Purposes, Elsevier Science Ltd, Amsterdam, The Netherlands (2007), 83-109.

A descriptor can be directly used as a predictor value (e.g. the total number of atoms of the test chemical compound). A predictor value can be created from a descriptor by removing the unit; for example: acetylsalicylic acid has a molar mass of 180.158 g/mol, the predictor value derived from the descriptor "molecular mass" can be 180.158.

There may be descriptors from which more than one predictor value is derived.

In a further step, a feature vector is generated which comprises the predictor values. The prediction model links the feature vectors of chemical compounds to their drug safety properties.

Information about clinical trials and whether certain chemical compounds have passed a clinical trial or have failed in a clinical trial due to a safety issue can be found in publically available databases or in databases licensed by third parties (e.g. TrialTrove, ClinicalTrials.gov, EORTC Clinical Trials Database, EU Clinical Trials Register (EU CTR), WHO International Clinical Trials Registry Platform, AACT Database, OpenTrials.net, etc.).

In a preferred embodiment, the prediction model is a machine learning prediction model which is trained with known chemical compounds (reference chemical compounds) which were subject to one or more non-oncological clinical trials and have either passed the one or more non-oncological clinical trials or have failed in one or more non-oncological clinical trials due to a safety issue.

The machine learning prediction model can be a classification model which classifies chemical compounds into two or more safety classes.

The machine learning prediction model can be a regression model which determines a safety score for a chemical compound the safety score representing the probability of the chemical compound failing in a non-oncological clinical trial due to a safety issue.

Methods of building a prediction model and training a machine learning prediction model can be found in the literature (see e.g.: Marco Gori: Machine Learning, A Constraint-Based Approach, Morgan Kaufmann Publishers, 2018, ISBN: 978-0-08-100659-7; Ethem Alpaydin: Introduction to Machine Learning, 3rd ed., The MIT Press 2014, ISBN: 978-0-262-02818-9; Shai Shalev-Shwartz and Shai Ben-David: Understanding Machine Learning - From Theory to Algorithms, Cambridge University Press 2104, ISBN: 978-1-107-05713-5).

The prediction model can be or comprise an artificial neural network. The present invention preferably uses an artificial neural network comprising at least three layers of processing elements: a first layer with input neurons (nodes), an Nth layer with at least one output neuron (node), and N-2 inner layers, where N is a natural number greater than 2.

In such a network, the output neuron(s) serve(s) to predict at least one safety score, the safety score representing a probability of a test chemical compound failing in a non-oncological clinical trial due to a safety issue. The input neurons serve to receive a feature vector of the test chemical compound as input values. Usually there is one input neuron for each predictor value of the feature vector.

The processing elements of the layers are interconnected in a predetermined pattern with predetermined connection weights therebetween. The network has been previously trained to determine a safety score from a feature vector. The training can be performed with a set of training data from a plurality of reference chemical compounds which passed one or more non-oncological clinical trials or failed in a clinical trial due to a safety issue.

When trained, the connection weights between the processing elements contain information regarding the relationship between the feature vector and the safety score.

Each network node represents a simple calculation of the weighted sum of inputs from prior nodes and a non-linear output function. The combined calculation of the network nodes relates the inputs to the output(s).

Training estimates network weights that allow the network to calculate (an) output value(s) close to the measured output value(s). A supervised training method can be used in which the output data is used to direct the training of the network weights. The network weights are initialized with small random values or with the weights of a prior partially trained network. The training data inputs are applied to the network and the output values are calculated for each training sample. The network output values are compared to the measured output values. A backpropagation algorithm is applied to correct the weight values in directions that reduce the error between measured and calculated outputs. The process is iterated until no further reduction in error can be made.

A cross-validation method can be employed to split the data into training and testing data sets. The training data set is used in the backpropagation training of the network weights. The testing data set is used to verify that the trained network generalizes to make good predictions. The best network weight set can be taken as the one that best predicts the outputs of the test data set. Similarly, varying the number of network hidden nodes and determining the network that performs best with the test data optimizes the number of hidden nodes.

Once the prediction model is trained and validated, it can be used to predict the safety properties of new chemical compounds which are herein referred as test chemical compounds.

In a first step, a test chemical compound is selected for which a prognosis shall be made whether the test chemical compound is safe (whether it is more likely that it will pass a clinical trial) or whether the test chemical compound is unsafe (whether it is more likely that it will fail in a clinical trial due to a safety issue).

In a further step, the chemical structure of the test chemical compound is determined. The chemical structure can be derived from the IUPAC (International Union of Pure an Applied Chemistry) name of the test chemical compound, or the chemical structure can be retrieved from a database of chemical compounds. The chemical structure can also be inputted by a user.

In a further step, a feature vector is generated which contains structural features of the test chemical compound. The feature vector is generated in the same way as the feature vectors of the reference chemical compounds used for building the prediction model: preferably, a plurality of descriptors is determined from the chemical structure of the test chemical compound, and then a plurality of predictor values is determined from the descriptors. Finally the feature vector is built from the predictor values.

In a further step, the feature vector is supplied to the prediction model. The prediction model is configured to determine a safety score from the feature vector. The safety score can be a binary variable (safe // unsafe), a ternary variable (safe // moderate // unsafe), a quaternary variable (approved drug // compound which passed phases I and II but failed in phase III // compound which passed phase I but failed in phase II // compound which failed in phase I), or any other gradual expression.

The safety score can also be a number in the range of 0 to 1 wherein the number 0 indicates that the test chemical compound will pass one or more non-oncological clinical trials, the number 1 indicates that the test chemical compound will fail in one or more non-oncological clinical trials, and any number between 0 and 1 indicates the probability of the test chemical compound failing in one or more non-oncological clinical trials.

The safety score can be outputted to a user.

It is also conceivable, that the predictor values which mainly led to the predicted safety score will be outputted to a user, so that the user is able to analyse which structural features might be responsible for the predicted outcome.

The prediction model is the heart of the present invention. It is usually implemented as a computer program which runs on a computer system.

A "computer" is a device for electronic data processing that processes data by means of programmable calculation rules. Such a device usually comprises a motherboard, that unit which comprises a processor for carrying out logical operations, as well as a peripheral.

In computer technology, "peripheral" refers to all devices which are connected to the computer and serve to control the computer and/or serve as input and output devices. Examples thereof are monitor (screen), printer, scanner, mouse, keyboard, drives, camera, microphone, loud speaker, etc. Internal connections and expansion cards are, too, considered to be peripherals in computer technology.

The computer together with its peripheral is herein referred to as "computer system".

Computers of today are in many cases divided into desktop PCs, portable PCs, laptops, notebooks, netbooks and tablet PCs and so-called handhelds (e.g. smartphones). The invention can be carried out with all these computers.

It is also conceivable that more than one computer system is used to run the invention. For example, the generation of descriptors from the chemical structure of the test chemical compound can be done by means of a first computer system and the determination of a safety score from the feature vector can be done by means of a second computer. Other configurations are also possible.

The invention will be more particularly elucidated below on the basis of figures and examples without the wish to restrict the invention to the features or combinations of features that are shown in the figures and examples.

The following are shown:

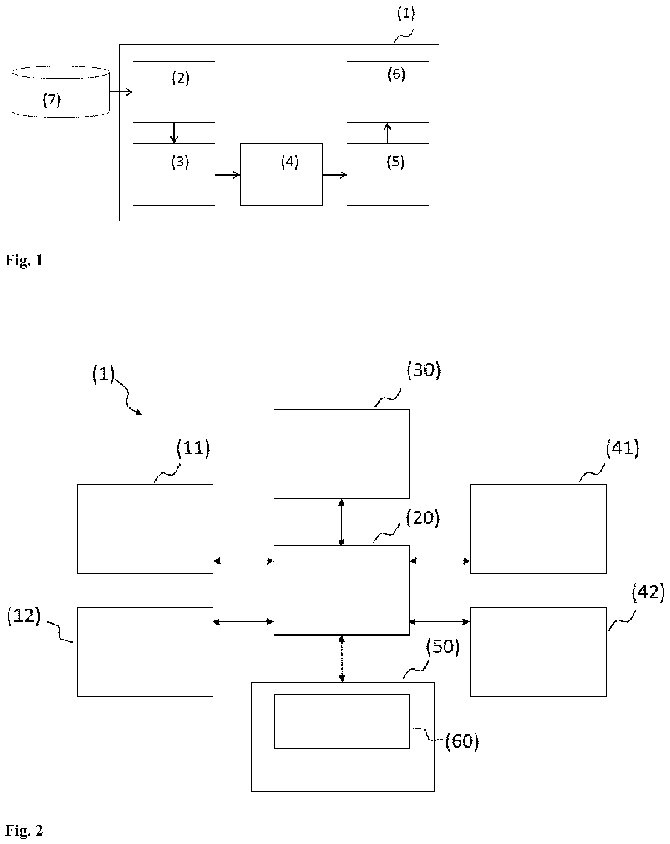

Fig. 1 illustrates a block diagram of an example computer system (1) to determine a safety score for a test chemical compound. The system (1) includes an input unit (2), a predictor value generation unit (3), a feature vector generation unit (4), a prediction unit (5), and an output unit (6).

The system (1) can receive information about the chemical structure of a test chemical compound via the input unit (2).

The system (1) can be connected to a database (7) in which chemical structures of chemical compounds are stored.

The system (1) can retrieve a chemical structure from the database (7) via the input unit (2).

It is also conceivable that a user enters the chemical structure of a test chemical compound via the input unit (2) into the system.

The input unit (2) can comprise one means or multiple means for inputting and/or for receiving information and/or data into/via the system (1). Means for inputting information preferably by the user are for example: keyboard, mouse, touch-sensitive screen (touchscreen), microphone and/or the like. Means for receiving information/data e.g. from a database are for example: network connection (LAN, WLAN), Bluetooth receiver and/or the like.

Information from which the chemical structure of the test chemical compound can be obtained are for example the name of the test chemical compound (e.g. the IUPAC name), an image of the chemical structure and/or the chemical structure as a file in a common format for chemical structures, including but not limited to Alchemy MOL, BioCad, TPL, CGM Metafile, ChemDraw, ChemPrint, Encap. Postscript, EPS w/WMF, HPGL, HyperChem HIN, ISIS/Daw, Kekule STR, MPG, PCModel, ROSDAL, SMILES, Softshell SCF, Sybyl MOL2, Sybyl Line Not and the like.

The predictor value generation unit (3) can retrieve the chemical structure of the test chemical compound from the input unit (2). Based on the structure, the predictor value generation unit (3) can determine a plurality of predictor values.

The computer system (1) can comprise a plurality of predictor value generation units, each being configured to determine one or more predictor values from the chemical structure.

It is also conceivable that one or more predictor values are inputted by a user via the input unit (2) into the computer system (1).

It is also conceivable that one or more predictor values are retrieved from the database (7).

The feature vector generation unit (4) can retrieve the plurality of predictor values from the predictor value generation unit (3). It is also conceivable that one or more predictor values are supplied from the input unit (2) to the feature vector generation unit (4).

The feature vector generation unit (4) can generate a feature vector from the plurality of predictor values.

The feature vector can be supplied from the feature vector generating unit (4) to the prediction unit (5).

The prediction unit (5) can determine a safety score from the feature vector. The prediction unit (5) can use a prediction model for determining the safety score.

The prediction model can be a machine learning prediction model which is trained to determine a safety score for a test chemical compound from a feature vector which is derived from the chemical structure of the test chemical compound.

The training of the prediction model can be done using the computer system according to the present invention.

The safety score is supplied from the prediction unit (5) to the output unit (6). The output unit (6) is configured to output the safety score to a user.

The output unit (6) can comprise one or more output means such as, for example, a screen, a printer, a loud speaker, a data-storage medium, a transmitting unit, a network access and the like.

Fig. 2 illustrates a computer system (1) according to some example implementations of the present disclosure. Generally, a computer system of exemplary implementations of the present disclosure may be referred to as a computer and may comprise, include, or be embodied in one or more fixed or portable electronic devices. The computer may include one or more of each of a number of components such as, for example, processing unit (20) connected to a memory (50) (e.g., storage device).

The processing unit (20) may be composed of one or more processors alone or in combination with one or more memories. The processing unit is generally any piece of computer hardware that is capable of processing information such as, for example, data, computer programs and/or other suitable electronic information. The processing unit is composed of a collection of electronic circuits some of which may be packaged as an integrated circuit or multiple interconnected integrated circuits (an integrated circuit at times more commonly referred to as a "chip"). The processing unit may be configured to execute computer programs, which may be stored onboard the processing unit or otherwise stored in the memory 50 (of the same or another computer).

The processing unit (20) may be a number of processors, a multi-core processor or some other type of processor, depending on the particular implementation. Further, the processing unit may be implemented using a number of heterogeneous processor systems in which a main processor is present with one or more secondary processors on a single chip. As another illustrative example, the processing unit may be a symmetric multi-processor system containing multiple processors of the same type. In yet another example, the processing unit may be embodied as or otherwise include one or more ASICs, FPGAs or the like. Thus, although the processing unit may be capable of executing a computer program to perform one or more functions, the processing unit of various examples may be capable of performing one or more functions without the aid of a computer program. In either instance, the processing unit may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions may be stored in memory, and executed by processing unit that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code (60) stored in the memory. It will also be understood that one or more functions, and combinations of functions, may be implemented by special purpose hardware-based computer systems and/or processing circuitry which perform the specified functions, or combinations of special purpose hardware and program code instructions.

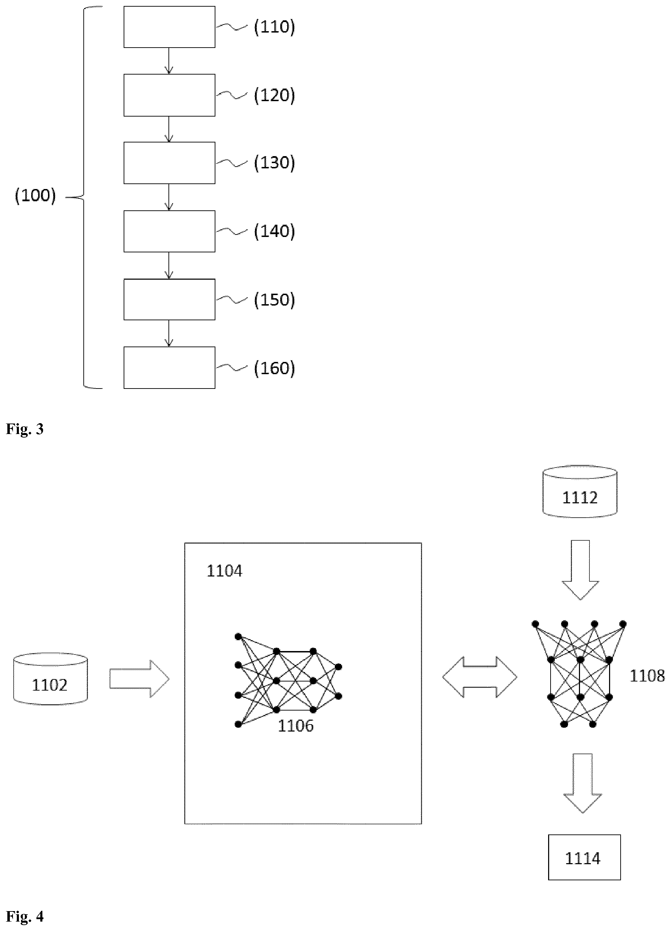

Fig. 3 shows schematically in the form of a flow chart one embodiment of the method (100) according to the invention.

The method (100) comprises the steps of:
(110) receiving a chemical structure of a test chemical compound,
(120) determining a plurality of predictor values from the chemical structure of the test chemical compound,
(130) generating a feature vector from the plurality of predictor values
(140) supplying the feature vector to a machine learning prediction model, the machine learning prediction model being trained with a first plurality of feature vectors and a second plurality of feature vectors, the first plurality of feature vectors being determined from chemical structures of reference chemical compounds which have failed in a non-oncological clinical trial due to a safety issue, the second plurality of feature vectors being determined from chemical structures of reference chemical compounds which passed a non-oncological clinical trial without any safety issue,
(150) receiving from the machine learning prediction model a safety score, the safety score representing a probability of the test chemical compound failing in a non-oncological clinical trial due to a safety issue.
(160) outputting the safety score.

Fig. 4 illustrates an exemplary training and deployment of an artificial neural network. Once a given network has been structured for a task the neural network is trained using a training dataset (1102).

To start the training process the initial weights may be chosen randomly or by pre-training using a deep belief network. The training cycle then be performed in either a supervised or unsupervised manner. Supervised learning is a learning method in which training is performed as a mediated operation, such as when the training dataset (1102) includes input paired with the desired output for the input, or where the training dataset includes input having known output and the output of the neural network is manually graded. The network processes the inputs and compares the resulting outputs against a set of expected or desired outputs. Errors are then propagated back through the system. The training framework (1104) can adjust the weights that control the untrained neural network (1106). The training framework (1104) can provide tools to monitor how well the untrained neural network (1106) is converging towards a model suitable to generating correct answers based on known input data. The training process occurs repeatedly as the weights of the network are adjusted to refine the output generated by the neural network. The training process can continue until the neural network reaches a statistically desired accuracy associated with a trained neural network (1108). The trained neural network (1108) can then be deployed to implement any number of machine learning operations. A feature vector of a test chemical compound (1112) can be inputted into the trained neural network (1108) to determine a safety score, the safety score representing a probability of the test chemical compound failing in a non-oncological clinical trial due to a safety issue.

## Claims

1. Method comprising the steps of:
- receiving a chemical structure of a test chemical compound,
- determining a plurality of predictor values from the chemical structure of the test chemical compound,
- generating a feature vector from the plurality of predictor values,
- supplying the feature vector to a machine learning prediction model, the machine learning prediction model being trained with a first plurality of feature vectors and a second plurality of feature vectors, the first plurality of feature vectors being determined from chemical structures of reference chemical compounds which have failed in a non-oncological clinical trial due to a safety issue, the second plurality of feature vectors being determined from chemical structures of reference chemical compounds which passed a non-oncological clinical trial without any safety issue,
- receiving from the machine learning prediction model a safety score, the safety score representing a probability of the test chemical compound failing in a non-oncological clinical trial due to a safety issue.
- outputting the safety score.

2. The method according to claim 1, wherein the feature vector is generated solely from the chemical structure of the test chemical compound.

3. The method according to claim 1 or 2, wherein the prediction model is trained and validated solely with data from past non-oncological clinical trials.

4. The method according to any one of claims 1 to 3, wherein the first plurality of feature vectors is determined solely from chemical structures of reference chemical compounds which have failed in a non-oncological phase I, phase II and/or phase III clinical trial due to a safety issue.

5. The method according to any one of claims 1 to 4, wherein the second plurality of feature vectors is determined solely from chemical structures of reference chemical compounds which, up to the time the prediction model is trained, have never failed in a non-oncological phase I, phase II, and phase III clinical trial due to a safety issue.

6. The method according to any one of claims 1 to 5, wherein the prediction model assigns the test chemical compound to one of a pre-defined number of classes, wherein the pre-defined number of classes comprises at least two classes, a first class comprising reference chemical compounds which failed in a phase I, phase II and/or phase III non-oncological clinical trial due to a safety issue and a second class comprising reference chemical compounds which passed a non-oncological phase I, phase II and/or phase III clinical trial.

7. The method according to any one of claims 1 to 5, wherein the prediction model assigns the test chemical compound to one of a pre-defined number of classes, wherein the pre-defined number of classes comprises at least two classes, a first class consisting of reference chemical compounds which failed in a non-oncological phase I or phase II or phase III clinical trial due to a safety issue and a second class consisting of reference chemical compounds which passed a phase I and phase II and phase III non-oncological clinical trial.

8. The method according to any one of claims 1 to 7, wherein the safety score is a number in the range of 0 to 1, wherein the number 0 indicates that the test chemical compound will pass one or more non-oncological clinical trials, the number 1 indicates that the test chemical compound will fail in one or more non-oncological clinical trials due to a safety issue, and any number between 0 and 1 indicates a probability of the test chemical compound failing in one or more non-oncological clinical trials.

9. The method according to any one of claims 1 to 8, wherein the prediction model is or comprises an artificial neural network.

10. A computer system comprising
- an input unit,
- one or more predictor value generation unit(s),
- a feature vector generation unit,
- a prediction unit, and
- an output unit,
wherein the input unit is configured to receive a chemical structure of a test chemical compound, wherein the one or more predictor value generation unit(s) is/are configured to determine a plurality of predictor values from the chemical structure of the test chemical compound,
wherein the feature vector generation unit is configured to generate a feature vector from the plurality of predictor values,
wherein the prediction unit is configured to determine a safety score from the feature vector using a machine learning prediction model,
- the machine learning prediction model being trained with a first plurality of feature vectors and a second plurality of feature vectors, the first plurality of feature vectors being determined from chemical structures of reference chemical compounds which have failed in a non-oncological clinical trial due to a safety issue, the second plurality of feature vectors being determined from chemical structures of reference chemical compounds which passed a non-oncological clinical trial without any safety issue,
- the safety score representing a probability of the test chemical compound failing in a non-oncological clinical trial due to a safety issue.
wherein the output unit is configured to output the safety score.

11. The computer system according to claim 10, wherein the machine learning prediction model is trained with a first plurality of feature vectors and a second plurality of feature vectors, the first plurality of feature vectors being determined solely from chemical structures of reference chemical compounds which have failed in a phase I or phase II or phase III non-oncological clinical trial due to a safety issue, the second plurality of feature vectors being determined from chemical structures of reference chemical compounds which passed a phase I and phase II and phase III non-oncological clinical trial without any safety issue.

12. The computer system according to claim 10 or 11, wherein the feature vector generation unit is configured to generate the feature vector solely from the plurality of predictor values from the chemical structure of the test chemical compound.

13. A computer program product comprising program code which can be loaded into a memory of a computer and causes the computer to execute the following steps:
- receiving a chemical structure of a test chemical compound,
- determining a plurality of predictor values from the chemical structure of the test chemical compound,
- generating a feature vector from the plurality of predictor values
- supplying the feature vector to a machine learning prediction model, the machine learning prediction model being trained with a first plurality of feature vectors and a second plurality of feature vectors, the first plurality of feature vectors being determined from chemical structures of reference chemical compounds which have failed in a non-oncological clinical trial due to a safety issue, the second plurality of feature vectors being determined from chemical structures of reference chemical compounds which passed a non-oncological clinical trial without any safety issue,
- receiving from the machine learning prediction model a safety score, the safety score representing a probability of the test chemical compound failing in a non-oncological clinical trial due to a safety issue.
- outputting the safety score.

14. The computer program product of claim 13, wherein the program code causes the computer to execute one or more steps of the method of any one of the claims 1 to 9.
